# FASCICULE DE BREVET EUROPEEN

(11) **EP 1 413 290 B1**
(45) Date de publication et mention de la délivrance du brevet: **17.05.2006**
(21) Numéro de dépôt: 03292437.5
(22) Date de dépôt: 02.10.2003
(51) Int. Cl.: A61Q 17/04, A61K 8/90, A61K 8/35

(54) **Procédé de photostabilisation de systèmes filtrants par des copolymères blocs et compositions cosmétiques photoprotectrices**
Verfahren zur Photostabilisierung von Sonnenschutzmitteln durch Blockcopolymere und Lichtschutzzusammensetzungen
Process for the photostabilisation of sunscreens through block-copolymers and cosmetic sunscreen compositions

(30) Priorité: 21.10.2002 FR 0213103
(43) Date de publication de la demande: 28.04.2004
(73) Titulaire: L'OREAL, 75008 Paris (FR)
(72) Inventeur: Chodorowski-Kimmes, Sandrine, 60300 Senlis (FR); Quinn, Francis, 75005 Paris (FR); Simonnet, Jean-Thierry, 94240 Cachan (FR)
(74) Mandataire: Dossmann, Gérard

(56) Documents cités:
- FR-A- 2 780 884
- US-A- 5 879 688
- US-A- 6 024 944

## Description

La présente invention est relative à un procédé de photostabilisation vis-à-vis du rayonnement UV de systèmes filtrants comprenant au moins un dérivé du dibenzoylméthane. Elle concerne également de nouvelles compositions, en particulier des compositions cosmétiques à usage topique.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres particulièrement intéressante est actuellement constituée par les dérivés du dibenzoylméthane, et notamment le 4-(tert.-butyl) 4'-méthoxy dibenzoylméthane, qui présentent un fort pouvoir d'absorption intrinsèque. Ces dérivés du dibenzoylméthane sont désormais des produits bien connus à titre de filtres actifs dans l'UV-A et sont notamment décrits dans les demandes de brevets FR-A-2326405 et FR-A-2440933, ainsi que dans la demande de brevet européen EP-A-0114607. Le 4-(tert.-butyl) 4'-méthoxy dibenzoylméthane est par ailleurs actuellement proposé à la vente sous la dénomination commerciale de « PARSOL 1789 » par la société HOFFMANN LAROCHE.

Toutefois, ces filtres, et notamment le Parsol® 1789, ou Butyl Methoxy Dibenzoylmethane (dénomination INCI), sont des produits relativement sensibles au rayonnement ultraviolet, c'est-à-dire qu'ils présentent une tendance à se dégrader sous l'action de ce dernier. Ainsi, ce manque de stabilité photochimique des filtres solaires face au rayonnement ultraviolet auquel ils sont par nature destinés à être soumis ne permet pas de garantir une protection constante durant une exposition solaire prolongée, de sorte que, de façon contraignante, des applications répétées à intervalles de temps réguliers et rapprochés doivent être effectuées par l'utilisateur pour obtenir une protection efficace de la peau contre les rayons UV.

Il est connu d'associer aux filtres solaires certains composés afin d'améliorer leur photostabilité. Ainsi, la demande EP 1093797 décrit un procédé pour améliorer la stabilité d'au moins un dérivé du dibenzoylméthane vis-à-vis du rayonnement UV, consistant à associer au dérivé du dibenzoylméthane un filtre organique insoluble sous forme micronisée dont la taille moyenne des particules varie de 0,01 à 2 µm.

La demande EP 0982310 décrit un procédé de photostabilisation d'un dérivé du dibenzoylméthane par un composé silanique ou organosilanique à fonction 2-hydroxybenzophénone.

La demande EP 1043966 décrit un procédé de photostabilisation d'un filtre solaire dérivé de 1,3,5-triazine par un composé à insaturation éthylénique.

La demande EP 1096916 décrit un procédé de photostabilisation d'un dérivé du dibenzoylméthane par un copolymère épaississant amphiphile qui n'est pas de type bloc.

Toutefois, les filtres solaires ainsi stabilisés présentent une résistance insuffisante à la dégradation par le rayonnement UV et la photostabilisation des filtres solaires photosensibles vis-à-vis du rayonnement UV constitue encore, à ce jour, un problème qui n'a pas encore été résolu de manière complètement satisfaisante.

Or, la Demanderesse vient de découvrir de façon surprenante qu'en associant à un système filtrant comprenant au moins un dérivé du dibenzoylméthane une quantité efficace d'au moins un copolymère amphiphile dibloc ou tribloc comportant au moins un bloc polymérique hydrophile non ionique et au moins un bloc polymérique hydrophobe, on parvient à photostabiliser le système filtrant.

La présente invention a donc pour objet un procédé pour photostabiliser vis-à-vis du rayonnement ultraviolet, en particulier vis-à-vis du rayonnement solaire, un système filtrant comprenant au moins un dérivé du dibenzoylméthane, ledit procédé étant essentiellement caractérisé par le fait qu'il consiste à associer au système filtrant comprenant au moins un dérivé du dibenzoylméthane une quantité efficace d'au moins un copolymère bloc (et notamment dibloc ou tribloc) amphiphile comportant au moins un bloc polymérique hydrophile non ionique et au moins un bloc polymérique hydrophobe.

Par quantité efficace, on entend la quantité de copolymère bloc nécessaire pour améliorer la photostabilité du système filtrant comprenant au moins un dérivé de dibenzoylméthane après une exposition UV contrôlée de 1h d'UV-A à 3,2 mW/cm² (soit 11,5 J/cm²) et de 1h d'UV-B de 0,22 mW/cm² (soit 0,79 J/cm²).

L'invention a également pour objet une composition cosmétique à usage topique comprenant, dans un milieu cosmétiquement acceptable, un système filtrant comprenant au moins un dérivé du dibenzoylméthane associé à une quantité efficace de certains copolymères blocs.

D'autres caractéristiques, aspects et avantages de l'invention apparaîtront à la lecture de la description détaillée qui va suivre.

Les copolymères blocs utilisés dans le procédé selon l'invention sont notamment des polymères blocs amphiphiles, préférentiellement non ioniques, diblocs ou triblocs, qui peuvent former au contact de l'eau des micelles, des particules de type vésicules (par exemple des liposomes) ou de type nanosphères, ou des phases cristal liquide lyotropes de type lamellaire, cubique (directe ou inverse) ou hexagonale (directe ou inverse) au contact de l'eau. Ils sont notamment de type dibloc (A-B) ou tribloc (A-B-A), A correspondant à un bloc polymérique hydrophile non ionique et B à un bloc polymérique hydrophobe. Le poids moléculaire des polymères peut être compris entre 1000 et 100.000 et le rapport A/B peut être compris entre 1/100 et 50/1.

Le bloc polymérique hydrophile non ionique peut être choisi parmi le polyoxyde d'éthylène (POE) et la polyvinylpyrrolidone (PVP).

Le bloc polymérique hydrophobe peut être choisi parmi le polystyrène, le poly(tert.-butylstyrène), le poly(méthylméthacrylate), le poly(éthylacrylate), le poly(butylacrylate), le poly(butylméthacrylate), le poly(vinylacétate), les polycaprolactones, les polycaprolactames, les polydiméthylsiloxanes, les polyoxydes d'alkylène en C₃-C₆, le poly(acide aspartique), le poly(acide lactique), le poly(acide glycolique), la polyleucine, les polybutadiène, les polyéthylènes, les polypropylènes, les polybutylènes.

Le copolymère bloc est choisi de préférence parmi les copolymères blocs suivants :
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthoxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on préfère tout particulièrement mettre en oeuvre le 4-(tert.-butyl)4'-méthoxydibenzoylméthane, ou Butyl Methoxy Dibenzoylmethane, notamment vendu sous la dénomination commerciale de « Parsol® 1789 » par la société HOFFMANN LAROCHE.

Le système filtrant peut en outre contenir un ou plusieurs composés choisis parmi les dérivés de 1,3,5-triazine, les dérivés de l'acide cinnamique et les hydroxybenzophénones aminosubstituées.

Les dérivés de 1,3,5-triazine conformes à l'invention préférentiels répondent à la formule générale suivante : 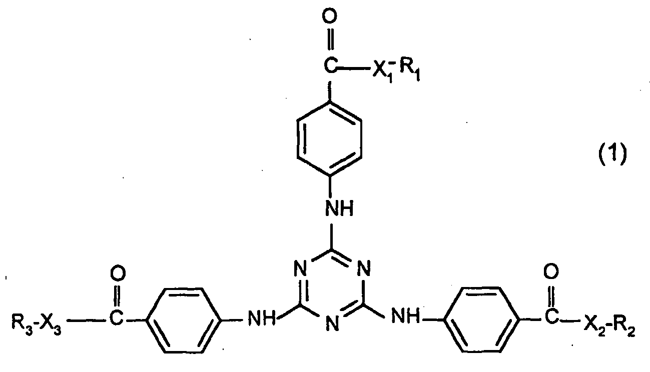 dans laquelle :
- X1, X2 et X3, identiques ou différents, représentent l'oxygène ou un radical -NR-;
- les radicaux R, identiques ou différents, désignent l'hydrogène ou un radical alkyle en C1-C18, linéaire ou ramifié, un radical cycloalkyle en C5-C12 pouvant être substitué par un ou plusieurs radicaux alkyle en C1-C4 ;
- R1, R2 et R3, identiques ou différents, sont choisis parmi : l'hydrogène; un radical alkyle linéaire ou ramifié en C1-C18 ; un radical cycloalkyle en C5-C12 éventuellement substitué par un ou plusieurs radicaux alkyle en C1-C4 ; un radical polyoxyéthyléné comprenant de 1 à 6 unités d'oxyde d'éthylène et dont le groupe OH terminal est méthylé ; un radical de formule (2), (3) ou (4) suivantes : 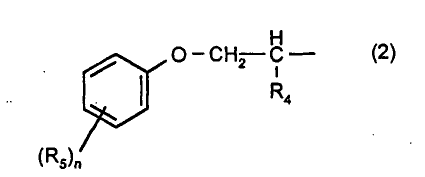 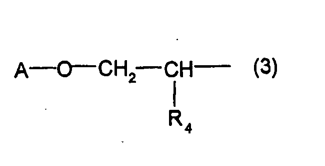 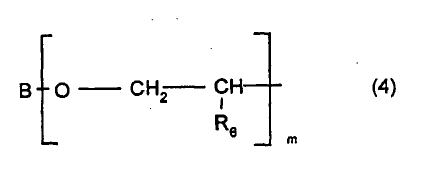
dans lesquelles :
- R4 est l'hydrogène ou un radical méthyle ;
- R5 est un radical alkyle en C1-C9 ;
- n est un nombre entier allant de 0 à 3 ;
- m est un nombre entier allant de 1 à 10 ;
- A est un radical alkyle en C4-C8 ou un radical cycloalkyle en C5-C8 ;
- B est choisi parmi : un radical alkyle linéaire ou ramifié en C1-C8 ; un radical cycloalkyle en C5-C8 ; un radical aryle éventuellement substitué par un ou plusieurs radicaux alkyle en C1-C4 ;
- R6 est l'hydrogène ou un radical méthyle ;

Une première famille préférée de dérivés de 1,3,5-triazine est celle, notamment décrite dans le document EP-A-0517104 (dont les enseignements sont, pour ce qui touche à la définition même de ces produits, totalement inclus à titre de référence dans la présente description) des 1,3,5-triazines répondant à la formule (1) ci-dessus et présentant l'ensemble des caractéristiques suivantes :
- X1, X2 et X3 sont identiques et représentent l'oxygène ;
- R1 est choisi parmi : un radical cycloalkyle en C5-C12 éventuellement substitué par un ou plusieurs radicaux alkyles en C1-C4 ; un radical de formule (2), (3) ou (4) ci-dessus dans lesquelles :
   - B est un radical alkyle en C1-C4 ;
   - R6 est le radical méthyle ;
   - R2 et R3, identiques ou différents, sont choisis parmi : l'hydrogène ; un radical alkyle linéaire ou ramifié en C1-C18 ; un radical cycloalkyle en C5-C12 éventuellement substitué par un ou plusieurs radicaux alkyle en C1-C4 ; un radical de formule (2), (3) ou (4) ci-dessus dans lesquelles :
      - B est un radical alkyle en C1-C4 ;
      - R6 est le radical méthyle.

Une deuxième famille préférée de dérivés de 1,3,5-triazine selon l'invention est celle, notamment décrite dans le document EP-A-0570838 (dont les enseignements sont, pour ce qui touche à la définition même de ces produits, totalement inclus à titre de référence dans la présente description) des 1,3,5-triazines répondant à la formule (1) et présentant l'ensemble des caractéristiques suivantes :
- X1 est l'oxygène ; X2 est le radical -NH- ou l'oxygène ;
- X3 est le radical -NH- ;
- R3 est choisi parmi : un radical alkyle linéaire ou ramifié en C1-C18 ; un radical cycloalkyle en C5-C12 éventuellement substitué avec un ou plusieurs radicaux alkyles en C1-C4 ;
- R1 est choisi parmi : l'hydrogène; un métal alcalin ; un radical ammonium; un radical de formule (4) ; un radical alkyle linéaire ou ramifié en C1-C18; un radical cycloalkyle en C5-C12 éventuellement substitué avec un ou plusieurs radicaux alkyles en C1-C4;
- si X2 est le radical -NH-, alors R2 est choisi parmi : un radical alkyle linéaire ou ramifié en C1-C18; un radical cycloalkyle en C5-C12 éventuellement substitué avec un ou plusieurs radicaux alkyles en C1-C4 ;
- si X2 est l'oxygène, alors R2 est choisi parmi l'hydrogène ; un radical de formule (4) ; un radical alkyle linéaire ou ramifié en C1-C18 ; un radical cycloalkyle en C5-C12 éventuellement substitué avec un ou plusieurs radicaux alkyles en C1-C4.

Une troisième famille préférée de dérivés de 1,3,5-triazine selon l'invention est celle, notamment décrite dans le document EP-A-0796851 dont les enseignements sont, pour ce qui touche à la définition même de ces produits, totalement inclus à titre de référence dans la présente description) des 1,3,5-triazines répondant à la formule (1) et présentant l'ensemble des caractéristiques suivantes :
- X1, X2 et X3 désignent simultanément -NR- ;
- les radicaux R, identiques ou différents, désignent l'hydrogène ou un radical alkyle en C1-C18, linéaire ou ramifié, un radical cycloalkyle en C5-C12 pouvant être substitué par un ou plusieurs radicaux alkyle en C1-C4 ;
- R1, R2 et R3, identiques ou différents, désignent l'hydrogène ou un radical R.

Une 1,3,5-triazine préférentielle est la 2,4,6-tris[p-(2'-éthylhexyl-1'-oxycarbonyl)-anilino]-1,3,5-triazine qui est un filtre connu en soi, actif dans l'UV-B, se présentant sous une forme solide, et qui est vendu notamment sous la dénomination commerciale de "UVINUL T150" par la Société BASF. Ce produit répond à la formule suivante : 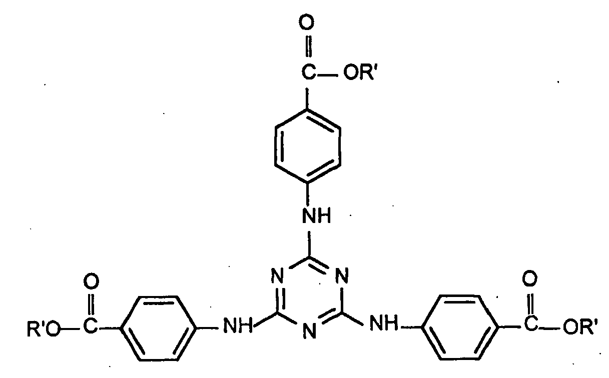 dans laquelle R' désigne un radical 2-éthylhexyle.

Un autre dérivé de 1,3,5-triazine selon l'invention particulièrement préféré est celui répondant à la formule (A) suivante : 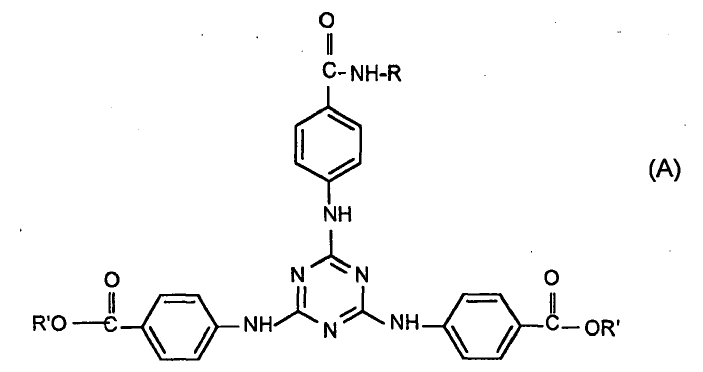 dans laquelle R' désigne un radical 2-éthylhexyle et R désigne un radical tert-butyle.

Parmi les dérivés de l'acide cinnamique mentionnés ci-dessus, on peut notamment citer, de manière non limitative les composés suivants, désignés par leur nom INCI, ainsi que leurs mélanges :
- Ethylhexyl Methoxycinnamate vendu notamment sous le nom commercial PARSOL MCX par HOFFMANN LAROCHE,
- Isopropyl Methoxycinnamate,
- Isoamyl Methoxycinnamate vendu sous le nom commercial NEO HELIOPAN E 1000 par HAARMANN ET REIMER,
- Cinoxate,
- DEA Methoxycinnamate,
- Diisopropyl Methylcinnamate,
- Glyceryl Ethylhexanoate Dimethoxycinnamate.

On citera également les silicones cinnamates portant éventuellement des substituants hydroxy, alkyle ou alcoxy en C₁-C₆, amino ou mono- ou di(C₁-C₆)alkyl amino.

Les hydroxybenzophénones aminosubstituées peuvent être choisies parmi les 2-hydroxybenzophénones aminées telles que celles décrites dans les demandes de brevets EP 1 046 391 et DE 1 00 12408, et notamment le 2-(4-diéthylamino-2-hydroxybenzoyl)benzoate de n-hexyle.

Les compositions selon l'invention comprennent un système filtrant comprenant au moins un dérivé du dibenzoylméthane et au moins un copolymère bloc choisi parmi les copolymères blocs suivants :
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

Le poids moléculaire des polymères peut être compris entre 1000 et 100.000 et le rapport en poids du ou des blocs polymériques hydrophiles non ioniques sur le ou les blocs polymériques hydrophobes peut être compris entre 1/100 et 50/1.

Le ou les dérivés du dibenzoylméthane peuvent être choisis parmi ceux décrits ci-dessus.

Le système filtrant présent dans les compositions selon l'invention peut en outre contenir un ou plusieurs composés choisis parmi les dérivés de 1,3,5-triazine, les dérivés de l'acide cinnamique et les hydroxybenzophénones aminosubstituées. Ces composés peuvent être choisis parmi ceux décrits ci-dessus.

Le rapport de concentrations en poids entre le système filtrant et le ou les copolymères blocs utilisés selon l'invention est de préférence compris entre 0,005 et 0,5.

De préférence, on veillera à choisir la quantité de copolymère bloc et de système filtrant de façon à ce que le système filtrant soit dispersé de manière homogène, c'est-à-dire sans qu'aucune recristallisation du dérivé de dibenzoylméthane ne soit observée après un jour à température ambiante.

L'association copolymère bloc/système filtrant peut être préparée en chauffant le mélange à une température nécessaire pour obtenir un mélange huileux isotrope. Ce mélange huileux peut être utilisé tel quel pour faire des films, ou bien être refroidi. Il pourra ensuite être dispersé dans un support cosmétique. Une autre mise en oeuvre possible, si le dérivé de dibenzoylméthane est thermosensible, est de solubiliser dans un solvant commun le copolymère bloc et le dérivé de dibenzoylméthane, puis d'évaporer ledit solvant. Le mélange obtenu pourra être traité comme précédemment.

Les compositions selon l'invention peuvent contenir de l'eau ou non. Dans le cas d'une composition hydratée, le rapport de concentrations en poids entre le système filtrant et le ou les copolymères blocs est notamment compris entre 0,005 et 0,2.

Le mode de mise en oeuvre du ou des copolymères blocs et du système filtrant à associer sera choisi par l'homme du métier en fonction de la nature chimique du polymère. A titre d'exemple, on citera la nanoprécipitation via un solvant miscible à l'eau, la dialyse ou l'hydratation directe du mélange copolymère bloc/système filtrant préalablement associés. La nanoprécipitation consiste à cosolubiliser dans un solvant organique miscible à l'eau et ayant un point d'ébullition inférieur à celui de l'eau, le ou les copolymères blocs et le système filtrant et d'introduire plus ou moins rapidement cette solution organique dans une phase aqueuse, sous agitation (par exemple à l'aide de barreau magnétique, pâles ou turbine). La formation des micelles ou particules est instantanée. Le solvant est ensuite évaporé.

La méthode par dialyse consiste à cosolubiliser dans un solvant organique miscible à légèrement miscible à l'eau et pouvant avoir un point d'ébullition supérieur à celui de l'eau, le ou les copolymères bloc et le système filtrant. Cette solution est introduite dans un sac de dialyse et dialysée contre de l'eau. L'eau de dialyse est régulièrement renouvelée, jusqu'à la dialyse complète du solvant. La même chose peut être envisagée avec des tensioactifs comme l'octyl glucoside, par exemple, à la place du solvant. C'est une solution aqueuse concentrée de tensioactifs qui solubilise le ou les copolymères et le composé lipophile pour avoir une solution limpide. Cette solution est ensuite dialysée jusqu' à élimination complète du tensioactif.

Une autre méthode, dite de Bangham, couramment utilisée pour faire des liposomes, peut être utilisée. Elle consiste à solubiliser dans un solvant organique, le ou les copolymères blocs et le système filtrant, puis à évaporer le solvant afin d'obtenir un mélange homogène du ou des copolymères blocs et du système filtrant. Au laboratoire, on pourra utiliser un évaporateur rotatif, industriellement un atomiseur, ou tout autre méthode permettant d'évaporer un solvant. Ce mélange peut ensuite être directement hydraté, sous agitation, par une solution aqueuse.

D'une façon générale, toutes les méthodes connues de l'homme du métier pour synthétiser des liposomes et des nanoparticules peuvent être utilisées.

Les compositions cosmétiques photostabilisées selon l'invention peuvent bien entendu contenir, outre le système filtrant, un ou plusieurs filtres solaires supplémentaires différents des précédents, hydrosolubles, liposolubles ou insolubles dans les solvants cosmétiques couramment utilisés. Ces filtres peuvent être choisis parmi les dérivés salicyliques, les dérivés du benzylidènecamphre, les dérivés de triazine tels que ceux décrits dans les brevets ou demandes de brevet US 4 367 390, EP 0 863 145, EP 0 517 104, EP 0 570 838, EP 0 796 851, EP 0 775 698, EP 0 878 469, EP 0 933 376 et EP 0 893 119, les dérivés de la benzophénone, les dérivés de β,β'-diphénylacrylate, les dérivés de phénylbenzimidazole, les dérivés anthraniliques, les dérivés d'imidazolines, les dérivés de méthylène-bis-(hydroxyphénylbenzotriazole) tels que ceux décrits dans les brevets ou demandes de brevet US 5 237 071, US 5 166 355, GB 2 303 549, DE 19726184 et EP 0 893 119, les dérivés de l'acide p-aminobenzoïque, les polymères hydrocarbonés filtres et les silicones filtres tels que ceux décrits notamment dans la demande WO 93/04665.

On peut citer à titre d'exemples de tels filtres solaires organiques additionnels non photosensibles actifs dans l'UV-A et/ou l'UV-B, les composés suivants désignés par leur nom INCI, ainsi que leurs mélanges :

### dérivés d'acide para-aminobenzoïque :

- PABA,
- Ethyl PABA,
- Ethyl Dihydroxypropyl PABA,
- Ethylhexyl Dimethyl PABA vendu notamment sous le nom commercial ESCALOL® 507 par ISP,
- Glyceryl PABA,
- PEG-25 PABA vendu sous le nom commercial UVINUL® P25 par BASF,

### dérivés salicyliques :

- Homosalate vendu sous le nom commercial EUSOLEX® HMS par RONA/EM INDUSTRIES,
- Ethylhexyl Salicylate vendu sous le nom commercial NEO HELIOPAN® OS par HAARMANN ET REIMER,
- Dipropyleneglycol Salicylate vendu sous le nom commercial DIPSAL® par SCHER,
- TEA Salicylate vendu sous le nom commercial NEO HELIOPAN® TS par HAARMANN ET REIMER,

### dérivés de β,β'-diphénylacrylate :

- Octocrylene vendu notamment sous le nom commercial UVINUL® N539 par BASF,
- Ethocrylene vendu notamment sous le nom commercial UVINUL® N35 par BASF ;

### dérivés de benzophénone :

- Benzophenone-1 vendue sous le nom commercial UVINUL® 400 par BASF,
- Benzophenone-2 vendue sous le nom commercial UVINUL® D-50 par BASF,
- Benzophenone-3 ou Oxybenzone vendue sous le nom commercial UVINUL® M-40 par BASF,
- Benzophenone-4 vendue sous le nom commercial UVINUL® MS-40 par BASF,
- Benzophenone-5,
- Benzophenone-6 vendue sous le nom commercial HELISORB® 11 par NORQUAY,
- Benzophenone-8 vendue sous le nom commercial SPECTRA-SORB® UV-24 par AMERICAN CYANAMID,
- Benzophenone-9 vendue sous le nom commercial UVINUL® DS-49 par BASF,
- Benzophénone-12,
- le 2-(4-diéthylamino-2-hydroxybenzoyl)-benzoate de n-hexyle,

### dérivés du benzylidène camphre :

- Benzylidene Camphor Sulfonic Acid fabriqué sous le nom MEXORYL® SL par CHIMEX,
- Camphor Benzalkonium Methosulfate fabriqué sous le nom MEXORYL® SO par CHIMEX,
- Terephthalylidene Dicamphor Sulfonic Acid fabriqué sous le nom MEXORYL® SX par CHIMEX,
- Polyacrylamidomethyl Benzylidene Camphor fabriqué sous le nom MEXORYL® SW par CHIMEX,
- 3-Benzylidene Camphor fabriqué sous le nom MEXORYL® SD par CHIMEX,
- 4-Methylbenzylidene Camphor vendu sous le nom EUSOLEX® 6300 par MERCK,

### dérivés de phénylbenzimidazole :

- Phenylbenzimidazole Sulfonic Acid vendu notamment sous le nom commercial EUSOLEX® 232 par MERCK,
- Benzimidazilate vendu sous le nom commercial NEO HELIOPAN® AP par HAARMANN ET REIMER,

### dérivés de triazine :

- Anisotriazine vendue sous le nom commercial TINOSORB S par CIBA GEIGY,
- la 2,4,6-tris-(4'-aminobenzalmalonate de diisobutyle)-s-triazine,

### dérivés de phénylbenzotriazole :

- Drometrizole Trisiloxane vendu sous le nom commercial SILATRIZOLE® par RHODIA CHIMIE,
- Methylene bis-Benzotriazolyl Tetramethylbutylphenol vendu sous forme solide sous le nom commercial MIXXIM® BB/100 par FAIRMOUNT CHEMICAL ou sous forme micronisée en dispersion aqueuse sous le nom commercial TINOSORB® M par CIBA SPECIALTY CHEMICALS,

### dérivés anthraniliques :

- Menthyl Anthranilate vendu sous le nom commercial NEO HELIOPAN® MA par HAARMAN ET REIMER,

### dérivés d'imidazolines :

- Ethylhexyl Dimethoxybenzylidene Dioxoimidazoline Propionate,

### dérivés du benzalmalonate :

- Polyorganosiloxane à fonctions benzalmalonate vendu sous le nom commercial PARSOL® SLX par HOFFMANN LAROCHE,

### dérivés de 4,4-diarylbutadiène :

- 1,1-dicarboxy (2,2'-diméthyl-propyl)-4,4-diphénylbutadiène,
Les filtres UV organiques plus particulièrement préférés sont choisis parmi les composés suivants :
- Ethylhexyl Salicylate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene,
- Benzimidazilate,
- Anisotriazine,
- 2,4,6-tris-(4'-aminobenzalmalonate de diisobutyle)-s-triazine,
- Ethylhexyl Triazone,
- Diethylhexyl Butamido Triazone,
- Methylene bis-Benzotriazolyl Tetramethylbutylphenol,
- Drometrizole Trisiloxane,
et leurs mélanges.

Les compositions selon l'invention peuvent également contenir des agents de bronzage et/ou de brunissage artificiel de la peau (agents autobronzants) tels que, par exemple, de la dihydroxyacétone (DHA).

Les compositions selon l'invention peuvent également contenir des pigments ou bien des nanopigments (taille moyenne des particules primaires : généralement entre 5 nm et 100 nm, de préférence entre 10 et 50 nm) d'oxydes métalliques enrobés ou non, comme par exemple, des nanopigments d'oxyde de titane (amorphe ou cristallisé sous forme rutile et/ou anatase) de fer, de zinc, de zirconium ou de cérium qui sont des agents photoprotecteurs bien connus en soi agissant par blocage physique (réflexion et/ou diffusion) du rayonnement UV. Des agents d'enrobage classique sont par ailleurs l'alumine et/ou le stéarate d'aluminium. De tels nanopigments d'oxydes métalliques, enrobés ou non enrobés, sont en particulier décrits dans les demandes de brevet EP-A-0 518 772 et EP-A-0 518 773.

Les compositions conformes à la présente invention peuvent contenir en outre des adjuvants cosmétiques classiques, notamment choisis parmi les corps gras, les solvants organiques, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline) ; végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination « Finsolv TN » par la société Finetex, le palmitate d'octyle, le lanolate d'isopropyle, les triglycérides dont ceux des acides caprique/caprylique), les esters et éthers gras éthoxylés ou propoxylés ; siliconées (cyclométhicone de préférence à 4 ou 5 atomes de silicium, polydiméthylsiloxane ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthylcellulose et les gommes de silicone comme par exemple un dérivé de polydiméthylsiloxane.

Les compositions de l'invention peuvent être préparées selon les techniques bien connues de l'homme du métier, en particulier celles destinées à la préparation d'émulsions du type huile-dans-l'eau ou eau-dans-huile.

Ces compositions peuvent se présenter en particulier sous forme de dispersion, notamment d'hydrodispersion, d'émulsion, simple ou complexe (H/E, E/H, H/E/H, ou E/H/E) telle qu'une crème, un lait ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

Les compositions cosmétiques photostabilisées selon l'invention peuvent être utilisées comme compositions protectrices de l'épiderme humain ou des cheveux contre les rayons ultraviolets, comme compositions antisolaires ou encore comme produits de maquillage.

Lorsque les compositions cosmétiques selon l'invention sont utilisées pour la protection de l'épiderme humain contre les rayons UV, ou comme composition antisolaire, elles peuvent se présenter sous forme d'huile gélifiée, de suspension ou de dispersion dans des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray, mais aussi sous forme de gel ou de lotion. Par lotion en entend une solution aqueuse, limpide à opalescente, sans huile.

Lorsque les compositions cosmétiques selon l'invention sont utilisées pour la protection des cheveux, elles peuvent se présenter sous forme de shampooing, de lotion, de gel, d'émulsion, de dispersion vésiculaire non ionique. Elles peuvent constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque les compositions cosmétiques selon l'invention sont utilisées comme produit de maquillage des cils, des sourcils ou de la peau, tel que crème de traitement de l'épiderme, fond de teint, bâton de rouge à lèvres, fard à paupières, fard à joues, mascara ou ligneur encore appelé « eye liner », elles peuvent se présenter sous forme solide ou pâteuse, anhydre ou aqueuse, comme des émulsions huile-dans-eau ou eau-dans-huile, des dispersions vésiculaires non ioniques ou encore des suspensions.

Les exemples suivants sont destinés à illustrer l'invention.

### Exemple 1

On prépare le mélange suivant :
- Copolymère bloc polystyrène/polyoxyéthylène vendu sous la dénomination Tegomer SE-1010 (société Goldschmidt, Polym) 1,20 g
- Parsol® 1789 0,22 g
On disperse le Parsol® 1789 dans le Tegomer SE-1010 en chauffant à 40°C pendant 60 minutes. On laisse le mélange revenir à température ambiante. Le mélange huileux est prêt à être employé.
Le mélange huileux est évalué sous forme d'une couche mince d'environ 10 microns et déposé sur une plaque de verre (lame de microscope), en présence d'oxygène.
On procède alors à une irradiation, en utilisant un UVIMETRE OSTRAM-Centra équipé d'une lampe au xénon, dans les conditions suivantes :
- Energie de la lampe UV-A : 22 J/cm²
- Temps d'irradiation du simulateur = 20 minutes
Ces conditions correspondent à environ une heure d'ensoleillement UV-A solaire.
La photodégradation est suivie par spectrophotométrie d'absorption.
On obtient les résultats suivants :
1) Le mélange est filmifiable et la qualité du film n'est pas altérée par l'irradiation.
2) On retrouve 100% du Parsol 1789 suite à cette exposition.
Le spectre d'absorption n'est pas modifié après irradiation

### Exemple 2

On prépare le mélange suivant :
On solubilise dans du dichlorométhane un copolymère bloc avec du Parsol® 1789 en excès. Le solvant est évaporé sous pression réduite à l'aide d'un évaporateur rotatif. Le film formé est alors hydraté avec de l'eau distillée, sous agitation, à une température comprise entre 20 et 80°C, pendant deux heures. Le taux de polymère dans l'eau est de l'ordre du pourcent.
24 heures plus tard, cette suspension est centrifugée afin de séparer le Parsol® 1789 qui n'a pas été encapsulé dans les micelles de polymère. Le surnageant, qui ne doit pas contenir de cristaux identifiables au microscope optique en lumière polarisée, est repris puis dosé par HPLC.
Le surnageant est ensuite irradié sous UV pendant une heure, l'intensité en UV-A étant de 3,2 mW/cm² et en UV-B de 0,22 mW/cm², soit respectivement 11,5 J/cm² et 0,79 J/cm², dans une plaque à 96 puits à raison de 100 µl de suspension par puit. Une plaque de quartz est posée dessus afin de limiter l'évaporation. Les suspensions sont ensuite à nouveau dosées par HPLC.
On obtient les résultats suivants :

| Copolymères blocs (dénominations et sociétés les commercialisant) | Nature chimique | Ratio A/B ou A/B/A | Pourcentage résiduel de Parsol® 1789 |
|---|---|---|---|
| SE 10-10 (Goldschmidt) | PS/POE | 1000/1000 | 81% |
| ME 10-10 (Goldschmidt) | Methyl Méthacrylate / POE | 1000/1000 | 91,50% |
| BE1010 (Goldschmidt) | Butyl Méthacrylate /POE | 1000/1000 | 85% |
| Plonon B208 (NOF) | POE / POB / POE | 3500/2000/3500 | 89% |
| P1044-SOE / P550-SOE | PS /POE | 3900/5100 | 91,50% |
| (80/20) (Polymer Source | | 12200/23900 | |
| P1503-SOE (Polymer Source) | PS/POE | 2300/3100 | 93,80% |
| P2351-EOCL (Polymer) Source | Polycaprolactone / POE | 800/5000 | 83% |
| P972-2EOCL (Polymer Source) | Polycaprolactone / POE | 1370/5000 | 85% |
| P1153-ETEO (Polymer Source) | Polyéthylène /POE | 5000/5900 | 60% |

| | | | |
|---|---|---|---|
| POE : polyoxyéthylène | | | |
| POB : polyoxybutylène | | | |
| PS : polystyrène | | | |

On observe une faible photodégradation du Parsol® 1789 avec les copolymères blocs utilisés selon l'invention.

### Exemple 3

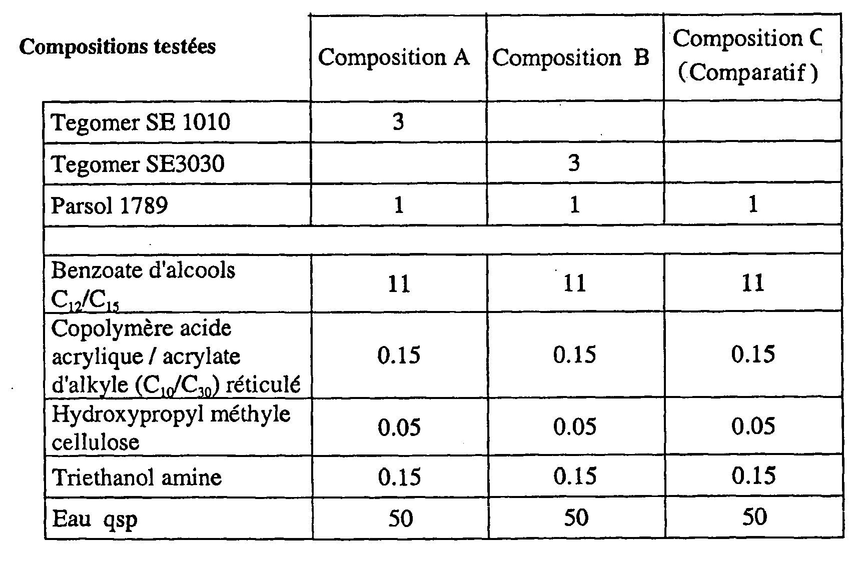

### Tests effectués

On mesure de la densité optique (DO) résiduelle après étalement à 0.75 mg/cm² des compositions sur des lames de quartz dépoli et exposition aux UV (Sun test Heraeus) pendant un temps équivalent à une énergie de 18 J/cm² mesurés en UVA.
Les plaques sont ensuite extraites à l'éthanol et on réalise un dosage spectrophotométrique à une longueur d'onde de 358 nm.

### Résultats

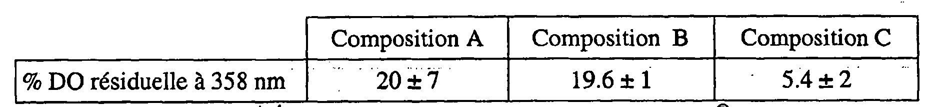

On observe une plus faible photodégradation du Parsol® 1789 avec les copolymères blocs utilisés selon l'invention que dans la composition C.

### Exemple 4

**Emulsion huile dans eau**

| *Phase Huileuse :* | | |
|---|---|---|
| Monostéarate de diglycérol | 2,0 | % |
| PEG-20 stearate | 1,5 | % |
| N Stearoyl L glutamique acide di sodique (Acyl glutamate HS 21 (Ajinomoto) | 0,5 | % |
| Huile de vaseline | 3 | % |
| Vaseline | 1 | % |
| Stéaryl Heptanoate | 3 | % |
| Huile d'amande d'abricot | 5 | % |
| Polyisobutène hydrogéné | 5 | % |
| Isocétyl palmitate | 2 | % |
| Silicone volatile | 5 | % |
| Vitamine E | 0,5 | % |
| Conservateur | 0,3 | % |

| Phase aqueuse 1 | | |
|---|---|---|
| Glycérol | 5 | % |
| Conservateurs | 1 | % |
| Eau distillée | Qsp 100 | % |

| Phase aqueuse 2 | | |
|---|---|---|
| Carbomer | 0,4 | % |
| Eau distillée | 15 | % |
| Conservateurs | 0,1 | % |
| Triéthanolamine | 0,4 | % |

| Phase aqueuse 3 | | |
|---|---|---|
| Suspension aqueuse de ME 10-10 à 20% dans l'eau, contenant 8% de Parsol 1789 | 10 | % |

Mode opératoire : la phase aqueuse 1 est introduite à 60°C dans la phase huileuse à 60°C, sous agitation très vive. On maintient la température et l'agitation pendant 30 minutes. La suspension est ensuite amenée à température ambiante.
La phase aqueuse 2 est alors dispersée à l'aide d'un disperseur non cisaillant. La phase aqueuse 3 (suspension de micelles) est ensuite introduite sous agitation légère.

## Revendications

1. Procédé de stabilisation vis-à-vis du rayonnement ultraviolet d'un système filtrant comprenant au moins un dérivé du dibenzoylméthane, **caractérisé en ce qu'**il consiste à associer audit système filtrant une quantité efficace d'au moins un copolymère amphiphile bloc comportant au moins un bloc polymérique hydrophile non ionique et au moins un bloc polymérique hydrophobe.

2. Procédé selon la revendication 1, **caractérisé en ce que** le poids moléculaire du copolymère bloc est compris entre 1000 et 100.000.

3. Procédé selon la revendication 1 ou 2, **caractérisé en ce que** le rapport en poids du ou des blocs polymériques hydrophiles non ioniques sur le ou les blocs polymériques hydrophobes est compris entre 1/100 et 50/1.

4. Procédé selon l'une quelconque des revendications précédentes, **caractérisé en ce que** le rapport de concentrations en poids entre le système filtrant et le ou les copolymères blocs est compris entre 0,005 et 0,5.

5. Procédé selon l'une quelconque des revendications précédentes, **caractérisé en ce que** le bloc polymérique hydrophile non ionique est choisi parmi le polyoxyde d'éthylène et la polyvinylpyrrolidone.

6. Procédé selon l'une quelconque des revendications précédentes, **caractérisé en ce que** le bloc polymérique hydrophobe est choisi parmi le polystyrène, le poly(tert.-butylstyrène), le poly(méthylméthacrylate), le poly(éthylacrylate), le poly(butylacrylate), le polybutylméthacrylate, le poly(vinylacétate), les polycaprolactones, les polycaprolactames, les polydiméthylsiloxanes, les polyoxydes d'alkylène en C₃-C₆, le poly(acide aspartique), le poly(acide lactique), le poly(acide glycolique), la polyleucine, le polybutadiène, le polyéthylène, le polypropylène, le polybutylène.

7. Procédé selon l'une quelconque des revendications précédentes, **caractérisé en ce que** le copolymère bloc est choisi de préférence parmi les copolymères blocs suivants :
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

8. Procédé selon l'une quelconque des revendications précédentes, **caractérisé en ce que** le ou les dérivés du dibenzoylméthane sont choisis dans le groupe constitué par :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5- diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert-butyl-4'-méthoxydibenzoylméthane.

9. Procédé selon la revendication 8, **caractérisé en ce que** le dérivé du dibenzoylméthane est le 4-tert.-butyl-4'-méthoxydibenzoylméthane.

10. Procédé selon l'une quelconque des revendications précédentes, **caractérisé en ce que** le système filtrant comprend en outre un ou plusieurs composés choisis parmi les dérivés de 1,3,5-triazine, les dérivés de l'acide cinnamique et les hydroxybenzophénones aminosubstituées.

11. Composition cosmétique comprenant dans un milieu cosmétiquement acceptable au moins un système filtrant comprenant au moins un dérivé du dibenzoylméthane, et au moins un copolymère bloc choisi parmi les copolymères blocs suivants :
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

12. Composition selon la revendication 11, **caractérisée en ce que** le poids moléculaire du copolymère bloc est compris entre 1000 et 100.000

13. Composition selon la revendication 11 ou 12, **caractérisée en ce que** le rapport en poids du ou des blocs polymériques hydrophiles non ioniques sur le ou les blocs polymériques hydrophobes est compris entre 1/100 et 50/1.

14. Composition selon l'une quelconque des revendications 11 à 13, **caractérisée en ce que** le rapport de concentrations en poids entre le système filtrant et le ou les copolymères blocs est compris entre 0,005 et 0,5.

15. Composition selon l'une quelconque des revendications 11 à 14, **caractérisée en ce qu'**elle est anhydre.

16. Composition selon l'une quelconque des revendications 11 à 14, **caractérisé en ce qu'**elle est hydratée.

17. Composition selon la revendication 16, **caractérisée en ce que** le rapport de concentrations en poids entre le système filtrant et le ou les copolymères blocs est compris entre 0,005 et 0,2.

18. Composition selon l'une quelconque des revendications 11 à 17, **caractérisée en ce que** le ou les dérivés du dibenzoylméthane sont choisis dans le groupe constitué par :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5- diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert-butyl-4'-méthoxydibenzoylméthane.

19. Composition selon la revendication 18, **caractérisée en ce que** le dérivé du dibenzoylméthane est le 4-tert.-butyl-4'-méthoxydibenzoylméthane.

20. Composition selon l'une quelconque des revendications 11 à 19, **caractérisée en ce que** le système filtrant comprend en outre un ou plusieurs composés choisis parmi les dérivés de 1,3,5-triazine, les dérivés de l'acide cinnamique et les hydroxybenzophénones aminosubstituées.

21. Composition selon l'une quelconque des revendications 11 à 20, **caractérisée en ce qu'**elle contient en outre un ou plusieurs filtres solaires supplémentaires différents des filtres cités dans les revendications précédentes et choisis parmi les dérivés salicyliques, les dérivés du dibenzylidènecamphre, les dérivés de la benzophénone, les dérivés de β,β'-diphénylacrylate, les dérivés de phénylbenzimidazole, les dérivés anthraniliques, les dérivés d'imidazolines, les dérivés de méthylène-bis-(hydroxyphénylbenzotriazole), les dérivés de l'acide p-aminobenzoïque, les polymères hydrocarbonés filtres et les silicones filtres.

22. Composition selon l'une quelconque des revendications 11 à 21, **caractérisée en ce qu'**elle contient en outre des nanopigments d'oxydes métalliques tels que l'oxyde de titane, l'oxyde de fer, l'oxyde de zinc, l'oxyde de zirconium et l'oxyde de cérium, éventuellement enrobés.

23. Composition selon l'une quelconque des revendications 11 à 22, **caractérisée en ce qu'**elle contient en outre au moins un agent de bronzage et/ou de brunissage artificiel de la peau.

24. Composition selon l'une quelconque des revendications 11 à 23, **caractérisée en ce qu'**elle contient en outre un ou plusieurs adjuvants de formulation choisis parmi les corps gras, les solvants organiques, les épaississants, les antioxydants, les opacifiants, les stabilisants, les agents anti-mousse, les parfums, les conservateurs, les charges, les séquestrants, les propulseurs, et les colorants.

## Claims

1. Process for stabilizing towards ultraviolet radiation a screening system comprising at least one dibenzoylmethane derivative, **characterized in that** it consists in combining the said screening system with an effective amount of at least one block amphiphilic copolymer comprising at least one nonionic hydrophilic polymer block and at least one hydrophobic polymer block.

2. Process according to Claim 1, **characterized in that** the molecular weight of the block copolymer is between 1000 and 100,000.

3. Process according to Claim 1 or 2, **characterized in that** the weight ratio of the nonionic hydrophilic polymer block(s) to the hydrophobic polymer block(s) is between 1/100 and 50/1.

4. Process according to any one of the preceding claims, **characterized in that** the weight concentration ratio between the screening system and the block copolymer(s) is between 0.005 and 0.5.

5. Process according to any one of the preceding claims, **characterized in that** the nonionic hydrophilic polymer block is chosen from polyethylene oxide and polyvinylpyrrolidone.

6. Process according to any one of the preceding claims, **characterized in that** the hydrophobic polymer block is chosen from polystyrene, poly(tert-butylstyrene), poly(methyl methacrylate), poly(ethyl acrylate), poly(butyl acrylate), poly(butyl methacrylate), poly(vinyl acetate), polycaprolactones, polycaprolactams, polydimethylsiloxanes, poly(C₃-C₆ alkylene oxides), poly(aspartic acid), poly(lactic acid), poly-(glycolic acid), polyleucine, polybutadiene, polyethylene, polypropylene and polybutylene.

7. Process according to any one of the preceding claims, **characterized in that** the block copolymer is preferably chosen from the following block copolymers:
- polystyrene/polyoxyethylene
- polymethyl methacrylate/polyoxyethylene
- polybutyl methacrylate/polyoxyethylene
- polyoxybutylene/polyoxyethylene
- polycaprolactone/polyoxyethylene
- polyethylene/polyoxyethylene
- polyoxyethylene/polyoxybutylene/polyoxyethylene.

8. Process according to any one of the preceding claims, **characterized in that** the dibenzoylmethane derivative(s) is(are) chosen from the group consisting of:
- 2-methyldibenzoylmethane,
- 4-methyldibenzoylmethane,
- 4-isopropyldibenzoylmethane,
- 4-tertbutyldibenzoylmethane,
- 2,4-dimethyldibenzoylmethane,
- 2,5-dimethyldibenzoylmethane,
- 4,4-diisopropyldibenzoylmethane,
- 4,4-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

9. Process according to Claim 8, **characterized in that** the dibenzoylmethane derivative is 4-tert-butyl-4'-methoxydibenzoylmethane.

10. Process according to any one of the preceding claims, **characterized in that** the screening system also comprises one or more compounds chosen from 1,3,5-triazine derivatives, cinnamic acid derivatives and amino-substituted hydroxybenzophenones.

11. Cosmetic composition comprising, in a cosmetically acceptable medium, at least one screening system comprising at least one dibenzoylmethane derivative, and at least one block copolymer chosen from the following block copolymers:
- polystyrene/polyoxyethylene
- polymethyl methacrylate/polyoxyethylene
- polybutyl methacrylate/polyoxyethylene
- polyoxybutylene/polyoxyethylene
- polycaprolactone/polyoxyethylene
- polyethylene/polyoxyethylene
- polyoxyethylene/polyoxybutylene/polyoxyethylene.

12. Composition according to Claim 11, **characterized in that** the molecular weight of the block copolymer is between 1000 and 100,000.

13. Composition according to Claim 11 or 12, **characterized in that** the weight ratio of the nonionic hydrophilic polymer block(s) to the hydrophobic polymer block(s) is between 1/100 and 50/1.

14. Composition according to any one of Claims 11 to 13, **characterized in that** the weight concentration ratio between the screening system and the block copolymer(s) is between 0.005 and 0.5.

15. Composition according to any one of Claims 11 to 14, **characterized in that** it is anhydrous.

16. Composition according to any one of Claims 11 to 14, **characterized in that** it is hydrated.

17. Composition according to Claim 16, **characterized in that** the weight concentration ratio between the screening system and the block copolymer(s) is between 0.005 and 0.2.

18. Composition according to any one of Claims 11 to 17, **characterized in that** the dibenzoylmethane derivative(s) is(are) chosen from the group consisting of:
- 2-methyldibenzoylmethane,
- 4-methyldibenzoylmethane,
- 4-isopropyldibenzoylmethane,
- 4-tertbutyldibenzoylmethane,
- 2,4-dimethyldibenzoylmethane,
- 2,5-dimethyldibenzoylmethane,
- 4,4-diisopropyldibenzoylmethane,
- 4,4-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

19. Composition according to Claim 18, **characterized in that** the dibenzoylmethane derivative is 4-tert-butyl-4'-methoxydibenzoylmethane.

20. Composition according to any one of Claims 11 to 19, **characterized in that** the screening system also comprises one or more compounds chosen from 1,3,5-triazine derivatives, cinnamic acid derivatives and amino-substituted hydroxybenzophenones.

21. Composition according to any one of Claims 11 to 20, **characterized in that** it also contains one or more additional sunscreens other than the screening agents mentioned in the preceding claims and chosen from salicylic derivatives, dibenzylidenecamphor derivatives, benzophenone derivatives, β,β'-diphenylacrylate derivatives, phenylbenzimidazole derivatives, anthranilic derivatives, imidazoline derivatives, methylenebis(hydroxyphenylbenzotriazole) derivatives, p-aminobenzoic acid derivatives, screening hydrocarbon-based polymers and screening silicones.

22. Composition according to any one of Claims 11 to 21, **characterized in that** it also contains nanopigments of metal oxides such as titanium oxide, iron oxide, zinc oxide, zirconium oxide and cerium oxide, which are optionally coated.

23. Composition according to any one of Claims 11 to 22, **characterized in that** it also contains at least one agent for artificially tanning and/or browning the skin.

24. Composition according to any one of Claims 11 to 23, **characterized in that** it also contains one or more formulation adjuvants chosen from fatty substances, organic solvents, thickeners, antioxidants, opacifiers, stabilizers, antifoams, fragrances, preserving agents, fillers, sequestering agents, propellants and dyes.

## Patentansprüche

1. Verfahren zur Stabilisierung eines Filtersystems, das mindestens ein Dibenzoylmethanderivat umfasst, gegenüber UV-Strahlung, **dadurch gekennzeichnet, dass** es darin besteht, in einer wirksamen Menge mindestens ein amphiphiles Blockcopolymer, das mindestens einen nichtionischen hydrophilen Polymerblock und mindestens einen hydrophoben Polymerblock enthält, mit dem Filtersystem zu kombinieren.

2. Verfahren nach Anspruch 1, **dadurch gekennzeichnet, dass** die Molmasse des Blockcopolymers im Bereich von 1.000 bis 100.000 liegt.

3. Verfahren nach Anspruch 1 oder 2, **dadurch gekennzeichnet, dass** das Gewichtsverhältnis des nichtionischen hydrophilen Polymerblocks oder der nichtionischen hydrophilen Polymerblöcke und des hydrophoben Polymerblocks oder der hydrophoben Polymerblöcke im Bereich von 1 / 100 bis 50/ 1 liegt.

4. Verfahren nach einem der vorhergehenden Ansprüche, **dadurch gekennzeichnet, dass** das auf das Gewicht bezogene Verhältnis der Konzentrationen des Filtersystems und des oder der Blockcopolymere im Bereich von 0,005 bis 0,5 liegt.

5. Verfahren nach einem der vorhergehenden Ansprüche, **dadurch gekennzeichnet, dass** der nichtionische hydrophile Polymerblock unter Polyethylenoxid und Polyvinylpyrrolidon ausgewählt ist.

6. Verfahren nach einem der vorhergehenden Ansprüche, **dadurch gekennzeichnet, dass** der hydrophobe Polymerblock unter Polystyrol, Poly(t-butylstyrol), Poly(methylmethacrylat), Poly(ethylacrylat), Poly(butylacrylat), Poly(butylmethacrylat), Poly(vinylacetat), Polycaprolactonen, Polycaprolactamen, Polydimethylsiloxanen, Polyalkylen(C₃₋₆)oxiden, Poly(asparaginsäure), Poly(milchsäure), Poly(glycolsäure), Polyleucin, Polybutadien, Polyethylen, Polypropylen und Polybutylen ausgewählt ist.

7. Verfahren nach einem der vorhergehenden Ansprüche, **dadurch gekennzeichnet, dass** das Blockcopolymer vorzugsweise unter den folgenden Blockcopolymeren ausgewählt ist:
- Polystyrol/polyoxyethylen
- Polymethylmethacrylat/polyoxyethylen
- Polybutylmethacrylat/polyoxyethylen
- Polyoxybutylen/polyoxyethylen
- Polycaprolacton/polyoxyethylen
- Polyethylen/polyoxyethylen
- Polyoxyethylen/polyoxybutylen/polyoxyethylen.

8. Verfahren nach einem der vorhergehenden Ansprüche, **dadurch gekennzeichnet, dass** das oder die Dibenzoylmethanderivat(e) unter den folgenden Verbindungen ausgewählt sind:
- 2-Methyldibenzoylmethan,
- 4-Methyldibenzoylmethan,
- 4-Isopropyldibenzoylmethan,
- 4-t-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Dimethoxydibenzoylmethan,
- 4-*t*-Butyl-4'-methoxydibenzoylmethan,
- 2-Methyl-5-isopropyl-4'-methoxydibenzoylmethan,
- 2-Methyl-5-*t*-butyl-4'-methoxydibenzoylmethan,
- 2,4-Dimethyl-4'-methoxydibenzoylmethan,
- 2,6-Dimethyl-4-*t*-butyl-4'-methoxydibenzoylmethan.

9. Verfahren nach Anspruch 8, **dadurch gekennzeichnet, dass** es sich bei dem Dibenzoylmethanderivat um das 4-t-Butyl-4'-methoxydibenzoylmethan handelt.

10. Verfahren nach einem der vorhergehenden Ansprüche, **dadurch gekennzeichnet, dass** das Filtersystem eine oder mehrere Verbindungen enthält, die unter den 1,3,5-Triazinderivaten, Zimtsäurederivaten und Amino-substituierten Hydroxybenzophenonen ausgewählt sind.

11. Kosmetische Zusammensetzung, die in einem kosmetisch akzeptablen Medium mindestens ein Filtersystem, das mindestens ein Dibenzoylmethanderivat umfasst, und mindestens ein Blockcopolymer enthält, das unter den folgenden Blockcopolymeren ausgewählt ist:
- Polystyrol/polyoxyethylen
- Polymethylmethacrylat/polyoxyethylen
- Polybutylmethacrylat/ polyoxyethylen
- Polyoxybutylen/polyoxyethylen
- Polycaprolacton/polyoxyethylen
- Polyethylen/polyoxyethylen
- Polyoxyethylen/polyoxybutylen/polyoxyethylen.

12. Zusammensetzung nach Anspruch 11, **dadurch gekennzeichnet, dass** die Molmasse des Blockcopolymers im Bereich von 1.000 bis 100.000 liegt.

13. Zusammensetzung nach Anspruch 11 oder 12, **dadurch gekennzeichnet, dass** das Gewichtsverhältnis des nichtionischen hydrophilen Polymerblocks oder der nichtionischen hydrophilen Polymerblöcke und des hydrophoben Polymerblocks oder der hydrophoben Polymerblöcke im Bereich von 1/100 bis 50/1 liegt.

14. Zusammensetzung nach einem der Ansprüche 11 bis 13, **dadurch gekennzeichnet, dass** das gewichtsbezogene Verhältnis der Konzentrationen des Filtersystems und des oder Blockcopolymere im Bereich von 0,005 bis 0,5 liegt.

15. Zusammensetzung nach einem der Ansprüche 11 bis 14, **dadurch gekennzeichnet, dass** sie wasserfrei ist.

16. Zusammensetzung nach einem der Ansprüche 11 bis 14, **dadurch gekennzeichnet, dass** sie hydratisiert ist.

17. Zusammensetzung nach Anspruch 16, **dadurch gekennzeichnet, dass** das auf das Gewicht bezogene Verhältnis der Konzentrationen des Filtersystems und des Blockcopolymers oder der Blockcopolymere im Bereich von 0,005 bis 0,2 liegt.

18. Zusammensetzung nach einem der Ansprüche 11 bis 17, **dadurch gekennzeichnet, dass** das oder die Dibenzoylmethanderivat(e) unter den folgenden Verbindungen ausgewählt sind:
- 2-Methyldibenzoylmethan,
- 4-Methyldibenzoylmethan,
- 4-Isopropyldibenzoylmethan,
- 4-t-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Dimethoxydibenzoylmethan,
- 4-t-Butyl-4'-methoxydibenzoylmethan,
- 2-Methyl-5-isopropyl-4'-methoxydibenzoylmethan,
- 2-Methyl-5-*t*-butyl-4'-methoxydibenzoylmethan,
- 2,4-Dimethyl-4'-methoxydibenzylmethan,
- 2,6-Dimethyl-4-t-butyl-4'-methoxydibenzoylmethan.

19. Zusammensetzung nach Anspruch 18, **dadurch gekennzeichnet, dass** es sich bei dem Dibenzoylmethanderivat um das 4-t-Butyl-4'-methoxydibenzoylmethan handelt.

20. Zusammensetzung nach einem der Ansprüche 11 bis 19, **dadurch gekennzeichnet, dass** das Filtersystem ferner mindestens eine oder mehrere Verbindungen enthält, die unter den 1,3,5-Triazinen, Zimtsäurederivaten und Amino-substituierten Hydroxybenzophenonen ausgewählt sind.

21. Zusammensetzung nach einem der Ansprüche 11 bis 20, **dadurch gekennzeichnet, dass** sie ferner ein oder mehrere ergänzende Sonnenschutzfilter enthält, die von den in den vorhergehenden Ansprüchen genannten Filtern verschieden sind und unter den Salicylsäurederivaten, Dibenzylidencampherderivaten, Benzophenonderivaten, β,β'-Diphenylacrylatderivaten, Phenylbenzimidazolderivaten, Anthranilderivaten, Imidazolinderivaten, Methylen-bis-(hydroxyphenylbenzotriazol)derivaten, *p*-Aminobenzoesäurederivaten, polymeren Filtern auf Kohlenwasserstoffbasis und Siliconfiltern ausgewählt sind.

22. Zusammensetzung nach einem der Ansprüche 11 bis 21, **dadurch gekennzeichnet, dass** sie ferner Nanopigmente von Metalloxiden, wie Titanoxid, Eisenoxid, Zinkoxid, Zirconiumoxid und Ceroxid enthält, die gegebenenfalls umhüllt sind.

23. Zusammensetzung nach einem der Ansprüche 11 bis 22, **dadurch gekennzeichnet, dass** sie ferner mindestens einen Stoff zur Bräunung und/oder künstlichen Braunfärbung der Haut enthält.

24. Zusammensetzung nach einem der Ansprüche 11 bis 23, **dadurch gekennzeichnet, dass** sie ferner einen oder mehrere Hilfsstoffe für die Formulierung enthält, die unter den Fettsubstanzen, organischen Lösungsmitteln, Verdickungsmitteln, Antioxidantien, Trübungsmitteln, Stabilisatoren, Schaumverhütungsmitteln, Parfums, Konservierungsmitteln, Füllstoffen, Maskierungsmitteln, Treibmitteln und Farbmitteln ausgewählt sind.
